# EUROPEAN PATENT APPLICATION

(11) **EP 1 941 890 A1**
(43) Date of publication of application: **09.07.2008**
(21) Application number: 06810469.4
(22) Date of filing: 19.09.2006
(51) Int. Cl.: A61K 35/50, A61P 7/06, A61P 9/04, A61P 9/10, A61P 25/16, A61P 25/28, A61P 35/00, A61P 35/02, A61P 43/00

(54) **HEMATOPOIETIC STEM CELL PROLIFERATOR**

(30) Priority: 16.09.2005 JP 2005271103
(71) Applicant: Yoshida, Kenji, Tokyo 104-0061 (JP)
(72) Inventor: Yoshida, Kenji, Tokyo 104-0061 (JP)
(74) Representative: HOFFMANN EITLE
(86) International application number: PCT/JP2006/318901
(87) International publication number: WO 2007/032566

(57) **Abstract**

Disclosed is a hematopoietic stem cell proliferation inducing agent which comprises a pulverized product of placenta-constituting cells as an active ingredient and can induce/proliferate a hematopoietic stem cell in the peripheral blood. Since the proliferation inducing agent can induce/proliferate a hematopoietic stem cell to increase the amount of hematopoietic stem cells in the peripheral blood, it becomes possible to ensure the increase in the amount of hematopoietic stem cells in the peripheral blood. The proliferation inducing agent can be used for the prevention and treatment of various diseases which are caused or believed to be caused by the decrease in the amount of hematopoietic stem cells (e.g., leukemia, malignant lymphoma, aplastic anemia, Alzheimer's disease, Parkinson's disease, dilated cardiomyopathy, myocardial infarction) and produces extremely small adverse side effects.

## Description

### TECHNICAL FIELD

The present invention relates to a hematopoietic stem cell proliferation inducing agent. More particularly, it relates to a hematopoietic stem cell proliferation inducing agent capable of inducing and proliferating hematopoietic stem cells in peripheral blood, and a manufacturing method of a pulverized product of placenta-constituting cells used in the proliferation inducing agent.

### BACKGROUND ART

Hematopoietic stem cells are immature cells produced in the bone marrow and having multi-differentiating capability, and as known well, they are cells differentiating into leucocytes, erythrocytes and platelets. Together with mesenchymal stem cells, they are called bone marrow stem cells, and are recently noticed as therapeutic materials at cell level for various organs and tissues.

At the present, hematopoietic stem cells are considered to be analogous to "cell surface markers/CD-34 positive cells." For identifying the hematopoietic stem cells, the CD-34 positive cells are searched from peripheral blood by combining a fluorescent antibody staining method and a flow cytometry. The CD-34 positive cells are known widely as safe graft materials not causing graft-versus-host disease. Usually, hematopoietic stem cells are present in the human bone marrow only by about 1 to 3% of whole monocytes, and are present in the peripheral blood only by about 0.1 to 0.5%. Even in umbilical cord blood, the existence ratio of hematopoietic stem cells is not so different from the level in the adult blood, being about 0.4 to 0.5%. Usually, the rate of hematopoietic stem cells in the peripheral blood tends to decrease along with aging, chronic regressive or chronic exhaustive disease. In the peripheral blood of a healthy adult person, the ratio of hematopoietic stem cells is 0.4%±0.05 (1SD) of all monocytes, and fluctuations are very small. According to the studies by the present inventor, the average hematopoietic stem cell ratio in peripheral blood in various disease groups was 0.2%±0.15 (1SD), and slight fluctuations were observed depending on the type and severity of diseases. In particular, in central nervous disease, myocardial disease, or infertility due to ovarian dysfunction, the hematopoietic stem cell ratio in peripheral blood was extremely low, and dropped to 0.1%.

At the present, for treatment of diseases such as hematopoietic tumors (for example, leukemia, malignant lymphoma, osteomyelodysplasia), aplastic anemia, solid tumors (for example, breast cancer), or disease due to enzyme deficiency, it has been attempted to transplant hematopoietic stem cells by dripping and infusing such hematopoietic stem cells. The infused hematopoietic stem cells are gradually introduced into the bone marrow, and produce various blood cells.

The hematopoietic stem cells used in such hematopoietic stem cell transplantation are obtained from the donor's bone marrow, from the donor's peripheral blood stem cells, or from the stored umbilical cord blood.

However, in the method of obtaining from the donor's bone marrow, the donor is generally anesthetized and is heavily stressed, and the bone marrow sampling amount is large. The donor, therefore, receives a heavy burden.

In the method of obtaining from the donor's peripheral blood stem cells, general anesthesia is not needed, and a proper amount of hematopoietic stem cells may be sampled. However, in order to increase the hematopoietic stem cells in the peripheral blood, a granulocyte colony stimulating factor (G-CSF) must be administered, and the G-CSF causes to increase granulocytes, which may not only lead to short-term side effects such as fever or general malaise, but also induce unexpected long-term side effects.

The stored umbilical cord blood is used only as auxiliary means.

Thus, in the hematopoietic stem cell transplantation, a large amount of hematopoietic stem cells is needed for treatment, and the donor's burden is significant, and it costs much expense, time and labor. Still more, the therapeutic effect by the transplantation is only temporary.

Recently it has been studied clinically to use hematopoietic stem cells produced from embryonic stem cells, but the use of embryonic stem cells for producing hematopoietic stem cells involves a serious ethical problem, and a wide consensus is required, but such consensus is not obtained globally.

As mentioned above, the hematopoietic stem cell transplantation involves various problems. Such problems of hematopoietic stem cell transplantation can be solved completely if hematopoietic stem cells in the patient's peripheral blood can be induced and proliferated without transplanting the hematopoietic stem cells.

More recently, it has been proved that the hematopoietic stem cells are not only differentiated and proliferated into various cells in the blood, but also that the hematopoietic stem cells induce cell fusion with nerve cells, myocardial cells and hepatocytes, thereby proliferating these cells (Nature, Vol. 425, 30 Oct. 2003, pp. 968-972). The hematopoietic stem cells flowing in peripheral blood have come to be understood to have various functions of repairing tissues and organs at cell level through the process of cell fusion, aside from the known functions. Therefore, to increase hematopoietic stem cells in peripheral blood ultimately leads to growth of nerve cells, myocardial cells and hepatocytes. Of course, all hematopoietic stem cells are stem cells of hemocytes, and it is expected to recover functions of blood cells, including the leukocytes playing the vital role in immune system.

The present inventor intensively repeated studies with the purpose of inducing and proliferating hematopoietic stem cells in peripheral blood without causing side effects in consideration of these problems, and discovered that the patient's hematopoietic stem cells can be auto-induced and proliferated by vaccine-like action possessed by a pulverized product of placenta-constituting cells isolated and purified from the placenta and/or placenta appendage (that is, cell membrane of placenta, intracellular organs and/or cell nucleus surface and/or internal antigen). Further, by administering an effective amount of the pulverized product of placenta-constituting cells, it is found to be effective to treat diseases caused by, or believe to be caused by, decrease of hematopoietic stem cells.

The invention is based on these findings, and it is hence an object thereof to present a hematopoietic stem cell proliferation inducing agent capable of increasing hematopoietic stem cells in peripheral blood by auto-induction and proliferation of the patient's own hematopoietic stem cells, and a manufacturing method of the pulverized product of placenta-constituting cells used when preparing the proliferation inducing agent.

### DISCLOSURE OF THE INVENTION

To solve the problems, the invention presents the hematopoietic stem cell proliferation inducing agent containing the pulverized product of placenta-constituting cells as an active ingredient. The pulverized product of placenta-constituting cells is preferred to be ground matter of placenta cell membrane, and the particle size of ground matter is preferably 1 to 200 µm, and the preferred dosage form is a freeze-dried preparation.

The manufacturing method of the invention is a method of the pulverized product of placenta-constituting cells used in the preparation, and includes the following steps:
(1) a step of washing and de-blooding placenta and/or placenta appendages;
(2) a step of pulverizing the resultant placenta and/or placenta appendages in an aqueous solvent, and centrifuging to obtain a solid matter; and
(3) a step of washing the solid matter with purified water.

### BEST MODE FOR CARRYING OUT THE INVENTION

The hematopoietic stem cell proliferation inducing agent of the invention contains the pulverized product of placenta-constituting cells as an active ingredient. The placenta used in the invention includes not only placenta body, but also placenta appendages such as umbilical cord, amnion, velamen (hereinafter the placenta and its appendages are collectively called placental materials).

As the placental materials, human placental materials are preferably used, but placental materials from other mammals (for example, swine, cow, rabbit and sheep) may be also used.

The pulverized product of placenta-constituting cells used in the hematopoietic stem cell proliferation inducing agent of the invention may be manufactured in various methods, and in particular the following method is preferred.

The manufacturing method of pulverized product of placenta-constituting cells of the invention is specifically described below.

Placental materials from normal delivery are desired, and infected materials are removed after inspecting for viruses such as HIV, HBV or HCV

First, the placental materials are washed and de-blooded with running water, and cut to square pieces of 2 to 3 cm, and further washed and de-blooded with tap water.

Next, the segmented placental materials are ground in purified water such as distilled water. Any ordinary grinding method is applicable, such as mixing by using an agitator. The mixing speed is not specified, but is generally about 8000 rpm. The amount of purified water may be properly adjusted, and usually about 1 part of purified water (by weight) is used in 1 part of the segmented placental materials. After mixing, the materials are centrifuged (3000 rpm, about 30 minutes). The separated placental materials are mixed and centrifuged again, and this operation is repeated until de-blooding is visibly complete, and the placental materials are ground uniformly.

The placental materials completely de-blooded and uniformly ground are presented to a next grinding process in an aqueous solvent by using a homogenizer or the like, by adding a surface active agent if necessary. By using the surface active agent, the placental materials are dispersed into individual cell levels, and cell structures are disintegrated, and it is easy to collect cell membranes and others. In the grinding process by using the surface active agent, proper amounts of purified water and surface active agents (for example, licorice extract, sodium glycyrrhizinate, ionic or nonionic surface active agent) are added to the placental materials, and mixed (8000 rpm, about 3 minutes). After mixing, the materials are centrifuged (3300 rpm, about 30 minutes), and solid matter is obtained. The obtained solid matter is presented to next washing process.

Meanwhile, to obtain the pulverized product of placenta-constituting cells mainly composed of the placenta cell membrane ground matter, the following centrifugal separation is operated. The separated solid matter is added to an aqueous solvent of which specific gravity is 1 or more, and the suspension is centrifuged, and the cell membrane components are separated. By using the aqueous solvent increased in specific gravity, the cell membrane components having lower specific gravity are suspended and floated on the superficial layer of the liquid, and can be separated from other cell components derived from the placenta and having relatively higher specific gravity. More specifically, the solid matter is collected from the layer floating in the superficial layer of the liquid after centrifugal separation. As the reagent for heightening the specific gravity, any material commonly used in this field may be used. Examples of the reagent may be glycerin, low molecular polyethylene glycol and the like. A specific example of aqueous solvent having a specific gravity of 1 or more is purified water containing glycerin by 5 to 50% (wt.%, same hereinafter), preferably 10 to 20 %. The mixing operation and the centrifugal separation operation are repeated until the suspended layer is no longer recognized in the superficial layer of the liquid. As a result of the repeated operation, placenta cell membrane pulverized matter is obtained.

The solid matter thus collected by centrifugal separation is washed by mixing with purified water and centrifugal separation. Next, as required, it is treated by a surface active agent. By the surface active agent treatment, the immune response of the pulverized product of placenta-constituting cells is enhanced, and the effect is further improved. Such surface active agent treatment can be done in the pharmaceutical preparation process.

In the operation of surface active agent treatment, to the washed solid matter, proper amounts of purified water and surface active agents (for example, licorice extract, sodium glycyrrhizinate) are added and mixed (8000 rpm, about 3 minutes), then centrifuged (3300 rpm, about 30 minutes), and the solid matter is collected.

Thus, the pulverized product of placenta-constituting cells treated with surface active agent as required is obtained. The obtained pulverized product of placenta-constituting cells is dried as required, and dried matter of pulverized product of placenta-constituting cells is obtained. The drying method is preferably moderate drying method in order to prevent degeneration, and a freeze-drying method is appropriate.

The pulverized product of placenta-constituting cells thus obtained may be pharmaceutically prepared by a conventional method. In a specific example, the solid matter obtained by centrifugal separation is pulverized by using a proper grinding machine. The pulverized powder is sieved through a screen having holes of 200 µm or less in diameter. As a result, the pulverized powder is uniform and extremely small in particle size. To the pulverized powder, purified water is added and the suspension is mixed uniformly, and poured into a vial. The vial is processed in vacuum freeze-drying process, and is sealed after vacuum freeze-drying process, so that the pharmaceutical preparation containing the pulverized product of placenta-constituting cells of the invention is obtained as milky white powder. The content of the solid matter per vial is not particularly specified, but is usually adjusted to about 50 to 100 mg.

This surface active agent treatment may be also operated immediately before pouring into the vial.

For pulverization, a ball mill or other usual grinding means may be used, and the particle size is about 1 to 200 µm, preferably about 5 to 50 µm. The effect may be further enhanced by pulverization.

The preparation is preferably sterilized, and the method of sterilization is not particularly specified, and non-heating sterilization by using electron ray or gamma-ray is preferred in order to prevent degeneration of the content.

In pharmaceutical preparation of hematopoietic stem cell proliferation inducing agent of the invention, preferably, a stabilizer is added, and examples of the stabilizer include albumin, globulin, gelatin, mannitol, glucose, dextran, ethylene glycol and the like. The preparation of the invention may contain additives necessary for pharmaceutical manufacturing, for example, a dissolving aid, an antioxidant, a pain-alleviating agent, an isotonic agent and the like. The preparation form may be preserved by removing moisture by freeze-storing or freeze-drying. The freeze-dried preparation is dispersed again before use by adding purified water for injection, 1 to 2% lidocaine solution (local anesthetic) and the like.

The effective dose and administration schedule of the hematopoietic stem cell proliferation inducing agent of the invention may be determined empirically, and are known for those skilled in the art. The route of administration of the preparation may be properly determined, it may be administered in the subcutaneous fatty tissues, the muscle and the like. The dose is properly adjusted depending on the symptom, age or body weight of the patient, being selected from a range of 1 mg to 10 mg/kg body weight, preferably 0.5 mg to 5 mg/kg body weight, more preferably 2 mg/kg body weight, and the dose is administered once or several times (2 or 3 times) a month. The site of administration is preferably positions abundant in fat tissues such as buttocks region.

The proliferation inducing agent of the invention may be administered as a conventional sustained-release agent known for those skilled in the art. Examples of sustained-release agent include microspheres (for example, nano-particle, micro particle, microcapsule, beads, liposome, composite emulsion).

The hematopoietic stem cell proliferation inducing agent of the invention is used in prevention and treatment of various diseases caused by, or believed to be caused by, decrease of hematopoietic stem cells. For example, it is used in prevention and treatment of diseases such as hematopoietic tumors (for example, leukemia, malignant lymphoma, osteomyelodysplasia), aplastic anemia, solid tumors (for example, breast cancer), and disease due to enzyme deficiency. It is also effective for prevention and treatment of central nerve diseases (for example, Alzheimer's disease, Parkinson's disease), heart disease (for example, dilated cardiomyopathy, myocardial infarction).

Interestingly enough, responsive reactions such as induction and proliferation of hematopoietic stem cells in peripheral blood caused by administration of the proliferation inducing agent of the invention are noted more obviously in elderly people or patients, rather than in young healthy people. This is estimated because young healthy people have the bone marrow functioning normally, and are provided sufficiently with hematopoietic stem cells in the body, whereas the elderly people or patients show a strong responsive reaction when the hematopoietic stem cells in the peripheral blood are lowered due to some cause or other, thereby inducing surging of the hematopoietic stem cells in the peripheral blood.

After the onset of surging of hematopoietic stem cells in a specific period after administration of the proliferation inducing agent of the invention, normal values are recovered gradually. The effect duration period varies with individuals, but generally by one treatment, the effect lasts for 2 weeks to about 2 months. In elderly people, weak effects are retained for a long period, but strong effects tend to be expressed for a short period in young subjects. This is considered because the hematopoietic stem cells are induced and proliferated by the pulverized product of placenta-constituting cells by way of vaccine-like action on the human immune system. That is, in the young people high in immune activity, it is estimated that a strong and short-term responsive reaction is likely to occur.

The hematopoietic stem cell proliferation inducing agent of the invention may be also used as animal medicine for other mammals than the human. In this case, by collecting placental materials from mammal animals, and treating similarly, the pulverized product of placenta-constituting cells is obtain, which is pharmaceutically prepared and administered to mammal animals. Applicable mammal animals include livestock animals (for example, swine, cattle, horse, sheep, rabbit) and companion animals (for example, dog, cat), and the same diseases as mentioned above may be treated.

### INDUSTRIAL APPLICABILITY

As shown in the Examples below, the hematopoietic stem cell proliferation inducing agent of the invention is capable of inducing and proliferating hematopoietic stem cells, and increasing the amount of hematopoietic stem cells in peripheral blood. Since the hematopoietic stem cells in peripheral blood are securely proliferated, various diseases caused by decrease of hematopoietic stem cells can be treated. As compared with the hematopoietic stem cell transplantation, the method of the invention is wider in the scope of effects and applications. Moreover, when prepared for induction of hematopoietic stem cells in peripheral blood by a hematopoietic stem cell induction treatment before and after various surgical operations, tissue and organ damages by surgical stresses can be controlled to a minimal extent. Considering from the clinical trial experiments, it may be clearly understood that proliferation of not only hematopoietic stem cells but also mesenchymal stem cells is strongly encouraged. However, as for the method of identifying and determining the mesenchymal stem cells in peripheral blood, any commonly acknowledge theory has not been established, and it cannot be directly proved at the present. However, seeing that myocardial diseases and other diseases estimated to be related with mesenchymal stem cells are cured completely, the hematopoietic stem cell proliferation inducing agent of the invention may be considered simultaneously as mesenchymal stem cell proliferation inducing agent. Summing up the discussion, the present invention is considered to provide bone marrow stem cell proliferation inducing agent.

The invention provides the effect only by injecting the hematopoietic stem cell proliferation inducing agent into the body (preferably by subcutaneous injection), and it is quite simple, and is extremely low in incidence of side effects.

Quite evidently, the hematopoietic stem cells are maternal cells of hemocytes. On the other hand, in cancer, sarcoma, and other malignant diseases, decline of function of immune system is observed at high rates, and decline of function of lymphocytes is very often observed. By hematopoietic stem cell induction method by administration of hematopoietic stem cell proliferation inducing agent of the invention, recovery of function of lymphocytes is observed, and hence the hematopoietic stem cell proliferation inducing agent of the invention is considered to induce not only induction and proliferation of hematopoietic stem cells in peripheral blood, but also recovery of function of the bone marrow.

According to the manufacturing method of the invention, the pulverized product of placenta-constituting cells used in the preparation can be obtained easily.

### Examples

The invention is more specifically described below by referring to Examples and Test Example, but the invention is not limited to these Examples alone.

### Example 1

Human placental materials were washed and de-blooded with running water. The placental materials were cut by a knife to square pieces of 2 to 3 cm, and were further washed and de-blooded. To 500 g of the segmented placenta materials (a portion of one placenta of ordinary size), 500 ml of distilled water was added, and mixed for 3 minutes. The mixing speed was about 8000 rpm. The mixture of placental materials and distilled water was centrifuged (3000 rpm, 30 minutes). The process of mixing and centrifuging was repeated 5 times. That is, the operation was repeated until the placental materials were de-blooded completely visibly, and ground uniformly.

To the placental materials de-blooded completely and ground uniformly, licorice extract was added as a surface active agent, and the materials were homogenized again (8000 rpm, 3 minutes). The homogenized placental materials were centrifuged (3300 rpm, 30 minutes).

To the separated placental materials, 10 to 20% glycerin solution was added, and the mixture was mixed and centrifuged (3300 rpm, 20 minutes). By this operation, cell membrane components having small specific gravity can be separated from other cell internal components having large specific gravity. Specifically, after centrifugal separation, the solid matter of suspended layer floating on the superficial layer of the liquid is scooped up. This mixing operation and centrifugal separation operation were repeated until the suspended layer is no longer recognized in the superficial layer of the liquid. The finally settling layer was discarded. By this operation, the pulverized product of placenta-constituting cells mainly composed of placenta cell membranes is obtained.

In order to remove glycerin, the collected solid matter was washed by mixing with distilled water and centrifugal separation. Distilled water and a proper amount of licorice extract as a surface active agent were added to the solid matter, and mixed and poured into a glass vial of 5 ml. The preparation was adjusted so that the solid content may be about 100 mg per vial.

The vial was subjected to freeze-dry, and a milky white powder of placenta cell membrane was obtained. The vial was closed by an aluminum seal. After sealing, the vial was sterilized by electron rays (irradiation dose 25 kG).

### Example 2

Human placental materials were washed and de-blooded with running water. The placental materials were cut by a knife to square pieces of 2 to 3 cm, and were further washed and de-blooded. To 500 g of the segmented placenta materials (a portion of one placenta of ordinary size), 500 ml of distilled water was added, and mixed for 3 minutes. The mixing speed was about 8000 rpm. The mixture of placental materials and distilled water was centrifuged (3000 rpm, 30 minutes). The process of mixing and centrifuging was repeated 5 times. That is, the operation was repeated until the placental materials were de-blooded completely visibly, and ground uniformly.

To the placental materials de-blooded completely and ground uniformly, licorice extract was added as a surface active agent, and the materials were homogenized again (8000 rpm, 3 minutes). The homogenized placental materials were centrifuged (3300 rpm, 30 minutes), and the solid matter was collected.

The collected solid matter was washed by mixing with distilled water and centrifugal separation. Then the solid matter was dried by freeze-drying. The dried solid matter was ground by a grinder, and the particle size was adjusted to 200 µm or less. To the ground solid matter, distilled water and a proper amount of licorice extract as a surface active agent were added and mixed, and poured into a glass vial of 5 ml. The preparation was adjusted so that the solid content may be about 100 mg per vial.

The vial was subjected to freeze-dry, and a milky white powder of pulverized product of placenta-constituting cells was obtained. The vial was closed by an aluminum seal. After sealing, the vial was sterilized by electron rays (irradiation dose 25 kG).

### Test Example 1

By obtaining the consent of six patients, the pulverized product of placenta-constituting cells preparation manufactured in Example 1 was re-dispersed in 1% lidocaine solution (local anesthetic), and was subcutaneously injected into the buttocks region of the patients (dose: 100 mg/patient).

After administration, the blood was sampled at time intervals, and the rate (%) of hematopoietic stem cells in monocytes in the peripheral blood was measured. The results are shown in Table 1.

As shown in Table 1, in patient numbers 1 to 4, in 14 days after administration, the rate of hematopoietic stem cells increased to 1.2 to 1.6%, and then gradually decreased, and induction of hematopoietic stem cells was recognized.

On the other hand, in patient numbers 5 and 6, sudden increase of hematopoietic stem cells was not noted, but the amount of hematopoietic stem cells tended to increase along with the passing of the time.

The difference in behavior between patient numbers 1 to 4 and patient numbers 5 and 6 is estimated as follows: in the former group, depending on the pathological conditions, the patient's body demanded hematopoietic stem cells, and induction and proliferation of hematopoietic stem cells seemed to be expressed prominently.

The preparation manufactured in Example 2 was similarly administered, and similar results were obtained.

**Table 1**

| | Days after administration | | |
|---|---|---|---|
| | Day 0 | Day 14 | Day 28 |
| Patient number 1 (female, 60 years) | 0.1 | 1.2 | 0.6 |
| Patient number 2 (male, 64 years) | 0.3 | 1.6 | 0.9 |
| Patient number 3 (female, 61 years) | 0.1 | 1.3 | 0.4 |
| Patient number 4 (male, 72 years) | 0.1 | 1.6 | 0.8 |
| Patient number 5 (male, 63 years) | 0.1 | 0.3 | 0.4 |
| Patient number 6 (female, 31 years) | 0.1 | 0.3 | 0.3 |

## Claims

1. A hematopoietic stem cell proliferation inducing agent containing a pulverized product of placenta-constituting cells as an active ingredient.

2. The hematopoietic stem cell proliferation inducing agent of claim 1, wherein the pulverized product of placenta-constituting cells is ground matter of placenta cell membrane.

3. The hematopoietic stem cell proliferation inducing agent of claim 1 or 2, wherein the particle size of pulverized product is 1 to 200 µm.

4. The hematopoietic stem cell proliferation inducing agent of claims 1 to 3, wherein the dosage form is a freeze-dried preparation.

5. A manufacturing method of a pulverized product of placenta-constituting cells comprising the following steps:
(1) a step of washing and de-blooding placenta and/or placenta appendages;
(2) a step of pulverizing the resultant placenta and/or placenta appendages in an aqueous solvent, and centrifuging to obtain a solid matter; and
(3) a step of washing the solid matter with purified water.

6. A treating method of diseases caused by, or believed to be caused by, decrease of hematopoietic stem cells, by administering an effective amount of a pulverized product of placenta-constituting cells.

7. The treating method of claim 6, wherein the diseases include hematopoietic tumors such as leukemia, malignant lymphoma and osteomyelodysplasia; aplastic anemia; solid tumors such as breast cancer; disease due to enzyme deficiency; central nerve diseases such as Alzheimer's disease and Parkinson's disease; and heart disease such as dilated cardiomyopathy and myocardial infarction.
